# EUROPEAN PATENT SPECIFICATION

(11) **EP 0 463 115 B1**
(45) Date of publication and mention of the grant of the patent: **04.09.1996**
(21) Application number: 90906035.2
(22) Date of filing: 23.01.1990
(51) Int. Cl.: C12P 21/02, C12N 15/18, C07K 14/61

(54) **ACETYLATED HETEROLOGOUS POLYPEPTIDES**
ACETYLIERTE HETEROLOGE POLYPEPTIDE
POLYPEPTIDES HETEROLOQUES ACETYLES

(30) Priority: 15.03.1989 US 323901
(43) Date of publication of application: 02.01.1992
(60) Divisional application: 96101997.3
(73) Proprietor: THE UPJOHN COMPANY, Kalamazoo, Michigan 49001 (US)
(72) Inventor: BRUNNER, David, Paul, Portage, MI 49081 (US); GARLICK, Robert, Lee, Augusta, MI 49012 (US); HARBOUR, Gary, Clyde, Kalamazoo, MI 49009 (US); LYLE, Stephen, Baker, Kalamazoo, MI 49001 (US); MOTT, John, Edward, Kalamazoo, MI 49004 (US); SHAFER, Jules, A., Ann Arbor, MI 48104 (US)
(74) Representative: Perry, Robert Edward
(86) International application number: US9000326
(87) International publication number: WO9010706

(56) References cited:
- CHEMICAL ABSTRACTS, vol. 108, no. 23, 06 June 1988, Columbus, OH (US); TEH, LIEW CHENG et al., p. 81, AN 198492b
- CHEMICAL ABSTRACTS, vol. 96, no. 9, 01 March 1982, Columbus, OHM (US); V. BLUMGRUND DE SATZ et al., p. 75, AN 63140y
- CHEMICAL ABSTRACTS, vol. 112, no. 1, 01 January 1990, Columbus, OH (US); LEE, FANG JEN S. et al., p. 428, AN 4283y
- FEBS LETTERS, vol. 59, no. 2, November 1975; M. YAGUCHI, pp. 217-220
- BIOCHEMISTRY & CELL BIOLOGY, vol. 64, 1986; S.R. ADAMSON et al., pp. 250-255
- CHEMICAL ABSTRACTS, vol. 107, no. 5, 03 August 1987, Columbus, OH (US); D. HOLZSCHU et al., p. 290, AN 35329u
- PROTEIN SCIENCE, vol. 3, 1995; pp. 1089-1097

## Description

### FIELD OF INVENTION

This invention is in the fields of microbiology and protein chemistry. More particularly, this invention relates to fermentation processes for producing recombinant heterologous polypeptides, particularly bovine somatotropin (rbSt) and a novel rbSt produced thereby.

### BACKGROUND OF THE INVENTION

Synthetic and chemically defined media for cultivating microorganisms are well known. Conventional nutrient media for cultivating bacteria have been used to grow recombinant bacteria that produce heterologous polypeptides. Decreasing the amount of expensive amino acids in growth media, while at the same time keeping expression of good quality heterologous polypeptides at a high level, is desirable. During fermentations in such media, production of novel acetylated analogs of heterologous polypeptides, for example rbSt, can be accomplished.

Naturally occurring bovine somatotropin (bSt), or growth hormone, can be purified from the pituitary glands of cattle as a mixture of heterogeneous proteins (Paladini, A. C. et al., 1983. CRC Reviews in Biochem., 15:25-56). Undefined electrophoretic heterogeneity is seen when native bovine pituitary extracts are fractionated by anion exchange chromatography (Hart, I.C. et al., 1984. Biochem. J., 218:573-581). Pituitary bovine somatotropin (pbSt) exists in isoforms which differ in isoelectric point (pI), with the most abundant form of pI 8.2. bSt is known to possess sites that can undergo deamidation, resulting in an increase in the number of lower pI forms which are seen as clusters of closely-spaced bands on isoelectric focusing at pI 7.0, 6.0, and 5.5 (Lewis, U.J., et al., 1970. Biochim. Biophys. Acta., 214:498-508; Secchi, C., et al., 1986. Int. J. Peptide Res. 28:298-306).

### INFORMATION DISCLOSURE

Acetylation of bovine somatotropin, principally at the ε-amino groups of lysine residues, results in a reduction in the positive charge of the protein due to the neutral nature of the acetyl moiety, thus producing bSt species with reduced pIs. Methods for chemical acetylation of bSt have been reported, but such means result in many acetylated products because of reactions with several other amino acids in addition to lysine (Oikawa, A., et al., 1967. Biochem. J., 104:947-952; De Satz, V.B. and J.A. Santome', 1981. Int. J. Peptide Protein Res. 18:492-499).

The acetylation of naturally occurring proteins is known. A dipeptide derived from Tobacco Mosaic virus was first shown to be modified by acetylation (Narita, K., 1958. Biochim. Biophys. Acta, 28:184-191). Subsequently, a large number of proteins, including ovalbumin, egg albumin, hemoglobins, and histones, have been shown to contain acetylated amino acids (Harris, J.I., 1959. Biochem. J. 71:445-459; Narita, K., 1961. Biochem. Biophys. Res. Comm. 5:160-164; Schroeder, W. A. et al., 1962. Biochim. Biophys. Acta, 63:532-534; Phillips, D.M.P., 1963. Biochem. J., 87:258-263). N^{α}-acetylation of proteins is widespread among eukaryotic and prokaryotic organisms and viruses (Driessen, H.P.C. et al., 1985. CRC Crit. Rev. Biochem., 18:281-325). For example, it has been suggested that 80% of the soluble proteins of Ehrlich ascites cells are N^{α}-acetylated (Brown, J.L. and W.K. Roberts, 1976. J. Biol. Chem., 251:1009-1014). N^{α}-acetylation appears to be a non-random process, as alanine and serine residues are the prime targets, whereas methionine, glycine, and aspartic acid are modified in only a few proteins, and several amino acids are not known to be N^{α}-acetylated (Wold, F., 1981. Ann. Rev. Biochem., 50:783-814).

Protein side-chain acetylation is another class of protein acetylation known to occur naturally, albeit less frequently than N^{α}-acetylations. The N^{ε}-acetylases are distinct from the N^{α}-acetylases, as they are specific for the ε-amino group of lysine and are located in the nucleus or cytosol, while the N^{α}-acetylases appear to be ribosome-associated (Sterner, R. et al., 1979. J. Biol. Chem., 254:11577-11583; Pestana, A. and H. C, Pitot, 1975. Biochemistry, 14:1397-1403; Pestana, A. and H.C. Pitot, 1975. Biochemistry, 14:1404-1412). However, both types of acetylases catalyze the transfer of acetyl from acetyl CoA to proteins (Wold, F., supra).

N^{ε}-acetylation of lysine residues was first shown to occur with histones H3 and H4 (Allfrey, V.G., et al., 1964. Proc. Nat. Acad. Sci. U.S.A., 51:78.6-794). In addition to histones, other DNA-binding proteins such as high-mobility group (HMG) proteins are N^{ε}-acetylated (Sterner, R., et al., supra).

Few proteins obtained from *Eseherichia coil* strains have been reported to be acetylated. The *E. coli* pyruvate dehydrogenase complex is acetylated at lipoyl residues which are linked to N^{ε}-amino groups of lysine residues on the protein (Adamson *et al* (1986), Biochem. Cell Biol. 64:250-255). Most reports concerning protein acetylation in *E. coli* have concentrated on acetylation of ribosomal proteins. N^{α}-acetylations of serine residues on *E. coli* ribosomal protein L7 (to produce protein L12) and alanine residues on ribosomal proteins S5 and S18 have been described (Brot *et al* (1972) Biochem. Biophys. Res. Commun. 49:673-679; Wittmann-Liebold *et al* (1978) FEBS Lett. 95:91-98; and Yaguchi (1976) FEBS Lett. 59:217-220).

### SUMMARY OF THE INVENTION

According to a first aspect of the present invention, an acetylated somatotropin is produced by growing E. coli transformed with a DNA sequence encoding the somatotropin on a fermentation medium having a concentration of amino-acids which, during or slightly preceding the somatotropin biosynthesis, is too low to repress the biosynthesis of, or to feedback inhibit, amino-acid biosynthesis enzymes.

According to a further aspect of the invention, a novel acetylated bovine somatotropin is acetylated only at lysine residues, among which is at least one of the amino-acid residues corresponding to the lysines at positions 157, 167, 171 and 180.

### DETAILED DESCRIPTION OF THE INVENTION

As used herein, "a low concentration of amino acids" means a concentration that is low enough such that it can be reduced by a microorganism cultured thereon to a "minimal concentration of amino acids", which is in turn defined as the concentration of exogenous amino acids which is too low to repress the biosynthesis of, or feedback inhibit effectively the activities of, amino acid biosynthetic enzymes. Such a reduction of the "low concentration of amino acids" to a "minimal concentration of amino acids" occurs during or slightly preceding the biosynthesis of the heterologous polypeptide. For example, with *E. coli* hosts and the rbSt polypeptide of the specific examples, the "low concentration of amino acids" is less than about 0.50 mM each, as derived from a 0.1% yeast extract supplement. The "low concentration of amino acids" is reduced to a "minimum concentration of amino acids" which is less than about 0.05 mM each.

Recombinant E. coli used in this invention is made by recombinant DNA techniques well known to those skilled in the art and set forth, for example, in *Molecular Cloning,* T. Maniatis, et al., Cold Spring Harbor Laboratory (1982) and A *Practical Guide to Molecular Cloning,* Perbal, B., John Wiley & Sons (1984).

The transformed E coli is cultivated in liquid nutrient medium. The medium comprises an excess of conventional nutrient materials that fulfil the cellular growth requirements of the microorganism, thereby enabling the microorganism to grow and multiply to a predetermined cellular density. This material includes sources of carbon, nitrogen and minerals such as sulfur, phosphorus, magnesium, potassium, copper, zinc, manganese, and iron. Amino-acids may be added in the form of protein hydrolysates usually made by subjecting naturally occurring proteinaceous materials, such as casein, soybean meal, lactalbumin, animal tissue, yeast cells and gelatin, to acid or enzymatic digestion. Mixtures of pure amino-acids may also be added.

Oxygen is also provided to the medium. To achieve maximum culture densities, the cultivation will usually be done in a way to enhance the area of the oxygen/liquid interface.

Important environmental factors affecting the cultivation include pH and temperature. The temperature will range between the minimum and maximum growth temperatures. Most bacteria exhibit maximum growth over a fairly narrow temperature range. For mesophilic bacteria, such as *E. coli,* the optimum temperature range is about 25°C to about 42°C, preferably about 37°C. Most organisms will tolerate hydrogen ion concentrations ranging over several pH units. For bacteria, such as *E. coli,* the tolerable pH lies in the range of about 6 to 8, with about 6.8 being preferred.

If expression of a gene encoding the acetylated somatotropin is under control of a repressible expression control sequence, one can specifically repress expression of that gene until a predetermined level of growth is reached by the cell culture by adding an appropriate repressor to the medium (e.g., tryptophan when expression is under control of the tryptophan promoter and operator).

After harvest, the cells are processed to recover the acetylated somatotropin. This normally involves disrupting the cells, separating crude acetylated somatotropin from bacterial proteins by one or more extraction steps, solubilizing the somatotropin (depending upon its hydrophobicity), oxidizing sulfhydryls to form proper disulfide bonds, when appropriate, and further purifying the somatotropin by ion-exchange chromatography, gel filtration, high performance liquid chromatography or other protein purification procedures.

We have been running fermentations to produce heterologous polypeptides by recombinant microorganisms, for example, rbSt in *E. coli* K-12 strains. These fermentations involve "low yeast extract" fermentation media containing 0.1% yeast extract, and effectively support over-expression of polypeptides in recombinant microorganisms.

Due to the molecular heterogeneity of somatotropins, the position numbers of amino acid residues of the various somatotropins may differ. The term "native mammalian somatotropin" includes these naturally occurring species. Chart 1 illustrates the specific amino acid sequence of one species of bSt. The numbering for other somatotropins may differ where analogs are involved. Those of ordinary skill in the art can readily locate corresponding amino acid sequences in alternative native mammalian somatotropins or their analogs.

pbSt exists in isoforms which differ in their respective isoelectric points. The most abundant form exhibits a pI of 8.2, with clusters of closely-spaced bands being evident on isoelectric focusing at pI 7.0, 6.0, and 5.5. RbSt was also found to contain lower pI species in clusters with pI of 7.0, 6.0, and 5.5. Incubation of rbSt in aqueous base such as 0.1M NH₄CO₃ (pH 10), causes a major shift to species with lower isoelectric points. Extensive structural analyses showed the modification following base treatment involved a conversion of the asparagine located at amino acid residue 99 (chart 1) to isoaspartic and aspartic acids (see WO-A-9008164).

Isolated rbSt which contained a replacement of the asparagine located at amino acid residue 99 (Asn99) (chart 1) with serine (Ser99), thus avoiding formation of isoaspartic acid, also contained rbSt species with pI of 7.0, 6.0, and 5.0. The pI 7.0 species of Ser99 rbSt accounted for approximately 30% of the total rbSt obtained. Independent structural analyses using purified pI 7.0 material of native rbSt (Asn99) showed at least four of the pI 7.0 species were formed by acetylation of lysine residues 157, 167, 171, and 180 (chart 1). Without such single acetylations, a native pI of 8.2 would be observed for Ser99 rbSt. Simultaneous acetylation of multiple lysine residues results in rbSt species exhibiting pI <7.0. The acetylation of other rbSt lysine residues (positions 30, 64, 70, 112, 114, 139, and 144, chart 1) has not been detected but may exist.

The purposeful acetylation of lysine residues during fermentations for protein overexpression makes available the synthesis of proteins with greater resistance to proteolytic degradation by trypsin and trypsin-like proteases and lysine-specific modifying reagents. Furthermore, as acetylation of lysine residues on the ε-amino group results in a reduction of the polypeptide's pI by one unit per acetylation, such polypeptides would be expected to be less susceptible to clearing by the kidneys from the circulation of a mammal administered the polypeptide. A lower pI would also be expected to increase the solubility of rbSt under the conditions used for isolation and formulation.

The acetylation of specific lysine residues during fermentations for the overexpression of rbSt was unexpected, and the mechanisms of such acetylation are not known. Prior to this invention there were no known fermentation means for obtaining rbSt with acetylations at lysine residues. Furthermore, there were no known means for preventing the acetylation of specific lysine residues during overexpression of rbSt.

Bacterial cultures: *E. coli* strain DU45 was used. This strain was derived from a wild-type strain of *E. coli* K-12 (ATCC e23716) by removal of the lambda prophage and the F plasmid and by introduction of the *rpoH112* allele. This strain was then transformed with plasmid pURA-99Ser-M4, a temperature-sensitive runaway replication vector, derived from plasmid pURA-4 and containing a gene encoding bSt (PCT patent application PCT/US 88/00328, incorporated herein by reference) having the asparagine at residue 99 (Chart 1) replaced with serine (see copending U.S. application S.N. 07/299,107).

Culture Media: The defined media were based upon medium E of Vogel and Bonner (Vogel, H. J. and D. M. Bonner, 1955. J. Biol. Chem., 218:97-106). The compositions and protocols for preparing the primary-seed and fermentation media are as follows:

| Primary-Seed Medium | |
|---|---|
| Components | Amount per Liter |
| Na(NH₄)HPO₄·H₂O | 10.90 g |
| K₂HPO₄ | 2.61 g |
| Citric Acid·H₂O | 2.10 g |
| MgSO₄·7H₂O | 0.99 g |
| (NH₄)₂SO₄ | 0.66 g |
| Yeast Extract | 10.40 g |
| Glycerol | 5.00 g |

For primary-seed cultures, the above components were hydrated with water (reverse-osmosis grade, R.O.) to 1000 ml, and divided into 300-ml volumes prior to sterilization. Seed medium was sterilized for 20 minutes at 121°C. Following sterilization, the seed medium was allowed to cool, and 0.5 ml sterile ampicillin (25 g/liter in R.O. water titrated to pH 8.0 with NaOH and filter sterilized, 0.45 µm) was added.

| Fermentation Medium | |
|---|---|
| Components | Amount per Liter |
| Na(NH₄)HPO₄·H₂O | 10.90 g |
| K₂HPO₄ | 2.61 g |
| Citric Acid (anhydrous) | 1.92 g |
| MgSO₄·7H₂O | 0.25 g |
| (NH₄)₂SO₄ | 0.66 g |
| Yeast Extract | 1.00 g |
| SAG4130 | 0.75 ml |

The fermentation medium was prepared by increasing the amounts of the above components 200-fold and hydrating them in 165 liters of R.O. water in a 250-liter fermentor. The fermentation medium was then sterilized for 20 minutes at 121°C (during sterilization, a medium volume increase of 20 liters was assumed, due to steam condensation). Following sterilization, the pH of the medium was checked to confirm a pH of 6.6 to 6.9. Upon cooling, two aseptic additions were made to complete the fermentation medium. The first addition consisted of a 200-ml aliquot of micronutrients (final concentrations: (NH₄)₆(MO₇)₂₄·4H₂O, 12 µM; H₃BO₃, 1.6 mM; Co- Cl₂·6H₂O, 120 µM; CuSO₄, 25.5 µM; MnCl₂·4H₂O, 319.4 µM; ZnSO₄·7H₂O, 40.1 µM) which had been sterilized by filtration (0.45 µm). The second addition consisted of 15 liters cerelose (8.24 kg cerelose q.s. to 14 liters with R.0. water and adjusted to pH 4.0 with H₂SO₄). The fermentation medium was adjusted to pH 7.2 with 25% NaOH prior to inoculation.

The low yeast extract fermentation medium contained 0.1% yeast extract for DU45 cultures.

### EXAMPLE Production of Acetylated rbSt During Low Yeast Extract Fermentations

Each primary-seed culture was inoculated with the contents of one culture ampoule (about 1 ml) and incubated at 28°C to a culture density of 0.7 to 0.8 A₅₅₀. Following incubation, each primary-seed culture was placed on ice prior to use for inoculation of the fermentors. All primary-seed cultures were supplemented with ampicillin to yield cultures containing essentially all plasmid-bearing bacteria. DU45 fermentations were initiated by inoculation with a 600-ml inoculum. Fermentor pH was controlled with anhydrous ammonia to maintain the pH at 7.2 to 7.4. Fermentor agitation and aeration rates were set at 320 rpm and 300 slm, at a backpressure of 10 psig. Cultures were grown at a permissive temperature of 27°C. DU45 fermentations were maintained at 27°C throughout, because plasmid runaway replication and rbSt synthesis were spontaneously induced (WO-A-8806186).

Culture aliquots were removed at various times from the primary seeds and fermentor and subjected to a series of analytical procedures to quantitate the culture density (absorbance at 550 nm), total bacterial dry weight, total rbSt concentration, glucose concentration, free ammonia concentration, acetate concentration, and microscopic identification and quantitation of inclusion bodies.

Purified rbSt preparations were derived from fermentation cultures of strain DU45 and subjected to isoelectric focusing (IEF) to quantitate the levels of acetylated rbSt (pI 7.0 vs. 8.2 species of Ser99-rbSt). The greater the level of pI 7.0 rbSt, the greater the fraction of total rbSt in the monoacetylated form. IEF was performed in 1 mm thick horizontal slab gels of a 5% T, 3% C composition and with a pH range of 3.5 to 9.5 (LKB PAGplate 1804-101). Gels were run in the absence of any denaturant at 1500 V maximum and at a constant power of 30W at 10°C. Gels were fixed for 30 minutes at room temperature in aqueous 11.5% (w/v) trichloroacetic acid, 3.5% (w/v) sulfosalicylic acid. Staining was performed for 10 minutes at 60°C with 0.1% (w/v) Coomassie Blue R250 in 25% ethanol:8% acetic acid. Gels were destained in 25% ethanol:8% acetic acid. Quantitation of rbSt species in IEF gels was done using a LKB laser densitometer.

Fermentation samples were obtained during the course of a low yeast extract fermentation and the levels of monoacetylated rbSt (pI 7.0 form of Ser99 rbSt) quantitated by IEF. The data in Table 1 show that the level of monoacetylated rbSt species in rbSt preparations ranges from a fractional percent of 24.9% to 34.7% during the course of the fermentation. Furthermore, these data show that the level of monoacetylated rbSt species is significant in early fermentation samples (e.g., 17 hours post-inoculation) and remains relatively constant throughout the duration of the fermentation.

**TABLE 1**

| LEVELS OF PI 8.2 AND 7.0 RBST SPECIES IN LOW YEAST EXTRACT FERMENTATION | | |
|---|---|---|
| Fermentation Sample¹ | Fractional Percent rbSt Species | |
| | pI 8.2 | pI 7.0 |
| 17 hrs | 71.4 | 28.6 |
| 19 hrs | 65.3 | 34.7 |
| 22 hrs | 72.9 | 27.1 |
| 26 hrs | 75.8 | 24.9 |
| 33 hrs | 75.0 | 25.0 |

| | | |
|---|---|---|
| ¹ Fermentation time in hours post-inoculation. Spontaneous induction of rbSt synthesis occurred at approximately 15 hours post-inoculation. | | |

CHART 1. Amino Acid Sequence of Bovine Somatotropin

10
ala phe pro ala met ser leu ser gly leu phe ala asn ala val

20 30
leu arg ala gln his leu his gln leu ala ala asp thr phe lys

40
glu phe glu arg thr tyr ile pro glu gly gln arg try ser ile

50 60
gln asn thr gln val ala phe cys phe ser glu thr ile pro ala

70
pro thr gly lys asn glu ala gln gln lys ser asp leu glu leu

80 90
leu arg ile ser leu leu leu ile gln ser trp leu gly pro leu

100
gln phe leu ser arg val phe thr asn ser leu val phe gly thr

110 120
ser asp arg val tyr glu lys leu lys asp leu glu glu gly ile

130
leu ala leu met arg glu leu glu asp gly thr pro arg ala gly

140 150
gln ile leu lys gln thr tyr asp lys phe asp thr asn met arg

160
ser asp asp ala leu leu lys asn try gly leu leu ser cys phe

170 180
arg lys asp leu his lys thr glu thr tyr leu arg val met lys

190
cys arg arg phe gly glu ala ser cys ala phe

## Claims (Claims for the following Contracting State(s): AT, BE, CH, DE, DK, FR, GB, IT, LI, LU, NL, SE)

1. A method for producing an acetylated somatotropin, which comprises growing E. coli transformed with a DNA sequence encoding the somatotropin on a fermentation medium having a concentration of amino-acids which, during or slightly preceding the somatotropin biosynthesis, is too low to repress the biosynthesis of, or to feedback inhibit, amino-acid biosynthesis enzymes.

2. A method according to claim 1, wherein the somatotropin is bovine somatotropin.

3. A method according to claim 1 or claim 2, wherein the concentration of amino-acids is less than 0.5 mM each.

4. Bovine somatotropin in which at least one of the amino-acid residues corresponding to the lysines at positions 157, 167, 171 and 180 are acetylated, and which is acetylated only at lysines.

5. Bovine somatotropin according to claim 4, acetylated only at position 157.

6. Bovine somatotropin according to claim 4, acetylated only at position 167.

7. Bovine somatotropin according to claim 4, acetylated only at position 171.

8. Bovine somatotropin according to claim 4, acetylated only at position 180.

## Claims (Claims for the following Contracting State(s): ES)

1. A method for producing an acetylated somatotropin, which comprises growing E. coli transformed with a DNA sequence encoding the somatotropin on a fermentation medium having a concentration of amino-acids which, during or slightly preceding the somatotropin biosynthesis, is too low to repress the biosynthesis of, or to feedback inhibit, amino-acid biosynthesis enzymes.

2. A method according to claim 1, wherein the somatotropin is bovine somatotropin.

3. A method according to claim 1 or claim 2, wherein the concentration of amino-acids is less than 0.5 mM each.

4. A method according to any preceding claim, wherein the somatotropin is bovine somatotropin in which at least one of the amino-acid residues corresponding to the lysines at positions 157, 167, 171 and 180 are acetylated, and which is acetylated only at lysines.

5. A method according to claim 4, wherein the somatotropin is bovine somatotropin, acetylated only at position 157.

6. A method according to claim 4, wherein the somatotropin is bovine somatotropin, acetylated only at position 167.

7. A method according to claim 4, wherein the somatotropin is bovine somatotropin, acetylated only at position 171.

8. A method according to claim 4, wherein the somatotropin is bovine somatotropin, acetylated only at position 180.

## Patentansprüche (Patentansprüche für folgende(n) Vertragsstaat(en): AT, BE, CH, DE, DK, FR, GB, IT, LI LU, NL, SE)

1. Verfahren zur Herstellung eines acetylierten Somatotropins durch Züchten von mit einer DNA-Sequenz mit Codierung für das Somatotropin transformierten E. coli auf einem Fermentationsmedium, das eine Aminosäurekonzentration aufweist, die - während oder kurz vor der Somatotropinbiosynthese - für eine Repression der Biosynthese von oder zur Rückkopplungshemmung von Aminosäurebiosyntheseenzymen zu gering ist.

2. Verfahren nach Anspruch 1, wobei es sich bei dem Somatotropin um Rindersomatotropin handelt.

3. Verfahren nach Anspruch 1 oder Anspruch 2, wobei die Aminosäurekonzentration jeweils unter 0,5 mMol liegt.

4. Rindersomatotropin, bei welchem mindestens einer der Aminosäurereste, die den Lysinen in den Positionen 157, 167, 171 und 180 entsprechen, acetyliert ist und welches lediglich an Lysin(positionen) acetyliert ist.

5. Rindersomatotropin nach Anspruch 4, das lediglich an Position 157 acetyliert ist.

6. Rindersomatotropin nach Anspruch 4, das lediglich an Position 167 acetyliert ist.

7. Rindersomatotropin nach Anspruch 4, das lediglich an Position 171 acetyliert ist.

8. Rindersomatotropin nach Anspruch 4, das lediglich an Position 180 acetyliert ist.

## Patentansprüche (Patentansprüche für folgende(n) Vertragsstaat(en): ES)

1. Verfahren zur Herstellung eines acetylierten Somatotropins durch Züchten von mit einer DNA-Sequenz mit Codierung für das Somatotropin transformierten E. coli auf einem Fermentationsmedium, das eine Aminosäurekonzentration aufweist, die - während oder kurz vor der Somatotropinbiosynthese - für eine Repression der Biosynthese von oder zur Rückkopplungshemmung von Aminosäurebiosyntheseenzymen zu gering ist.

2. Verfahren nach Anspruch 1, wobei es sich bei dem Somatotropin um Rindersomatotropin handelt.

3. Verfahren nach Anspruch 1 oder Anspruch 2, wobei die Aminosäurekonzentration jeweils unter 0,5 mMol liegt.

4. Verfahren nach einem der vorhergehenden Ansprüche, wobei es sich bei dem Somatotropin um Rindersomatotropin handelt, bei welchem mindestens einer der Aminosäurereste, die den Lysinen in den Positionen 157, 167, 171 und 180 entsprechen, acetyliert ist und welches lediglich an Lysin(positionen) acetyliert ist.

5. Verfahren nach Anspruch 4, wobei das Somatotropin aus lediglich in Position 157 acetyliertem Rindersomatotropin besteht.

6. Verfahren nach Anspruch 4, wobei das Somatotropin aus lediglich in Position 167 acetyliertem Rindersomatotropin besteht.

7. Verfahren nach Anspruch 4, wobei das Somatotropin aus lediglich in Position 171 acetyliertem Rindersomatotropin besteht.

8. Verfahren nach Anspruch 4, wobei das Somatotropin aus lediglich in Position 180 acetyliertem Rindersomatotropin besteht.

## Revendications (Revendications pour l'(les) Etat(s) contractant(s) suivant(s): AT, BE, CH, DE, DK, FR, GB, IT, LI, LU, NL, SE)

1. Procédé de production d'une somatotropine acétylée qui comprend la prolifération d'E. coli. transformé avec une séquence d'ADN codant pour la somatotropine dans un milieu de fermentation dont la concentration en acides aminés, pendant ou au moment précédant de peu la biosynthèse de somatotropine, est trop faible pour réprimer la biosynthèse d'enzymes responsables de la biosynthèse d'acides aminés ou pour inhiber ces enzymes de manière rétroactive.

2. Procédé selon la revendication 1, dans lequel la somatotropine est la somatotropine d'origine bovine.

3. Procédé selon la revendication 1 ou la revendication 2, dans lequel la concentration de chacun des acides aminés est inférieure à 0,5 mM.

4. Somatotropine bovine dans laquelle au moins l'un des résidus d'acides aminés correspondant aux lysines en position 157, 167, 171 et 180 est acétylé, et qui est acétylée uniquement au niveau des lysines.

5. Somatotropine bovine selon la revendication 4, acétylée uniquement en position 157.

6. Somatotropine bovine selon la revendication 4, acétylée uniquement en position 167.

7. Somatotropine bovine selon la revendication 4, acétylée uniquement en position 171.

8. Somatotropine bovine selon la revendication 4, acétylée uniquement en position 180.

## Revendications (Revendications pour l'(les) Etat(s) contractant(s) suivant(s): ES)

1. Procédé de production d'une somatotropine acétylée qui comprend la prolifération d'E. coli. transformé avec une séquence d'ADN codant pour la somatotropine dans un milieu de fermentation dont la concentration en acides aminés, pendant ou au moment précédant de peu la biosynthèse de somatotropine, est trop faible pour réprimer la biosynthèse d'enzymes responsables de la biosynthèse d'acides aminés ou pour inhiber ces enzymes de manière rétroactive.

2. Procédé selon la revendication 1, dans lequel la somatotropine est la somatotropine d'origine bovine.

3. Procédé selon la revendication 1 ou la revendication 2, dans lequel la concentration de chacun des acides aminés est inférieure à 0,5 mM.

4. Procédé de production selon l'une quelconque des revendications précédentes, dans lequel la somatotropine est la somatotropine d'origine bovine dans laquelle au moins l'un des résidus d'acides aminés correspondant aux lysines en position 157, 167, 171 et 180 est acétylé, et qui est acétylée uniquement au niveau des lysines.

5. Procédé selon la revendication 4, dans lequel la somatotropine est la somatotropine bovine, acétylée uniquement en position 157.

6. Procédé selon la revendication 4, dans lequel la somatotropine est la somatotropine bovine, acétylée uniquement en position 167.

7. Procédé selon la revendication 4, dans lequel la somatotropine est la somatotropine bovine, acétylée uniquement en position 171.

8. Procédé selon la revendication 4, dans lequel la somatotropine est la somatotropine bovine, acétylée uniquement en position 180.
